(19) Europäisches Patentamt
European Patent Office
Office européen des brevets

(11) **EP 3 178 920 A1**

(12) **EUROPEAN PATENT APPLICATION**
published in accordance with Art. 153(4) EPC

(43) Date of publication:
**14.06.2017 Bulletin 2017/24**

(21) Application number: **15830237.2**

(22) Date of filing: **06.08.2015**

(51) Int Cl.:
*C12N 1/20* [(2006.01)]        *A01N 63/00* [(2006.01)]

(86) International application number:
**PCT/CL2015/050031**

(87) International publication number:
**WO 2016/019480 (11.02.2016 Gazette 2016/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **08.08.2014   CL 20142101**

(71) Applicant: **Universidad De Santiago De Chile CP 9170022, Estación Central Santiago (CL)**

(72) Inventors:
- **SANTIAGO OLMEDO, Roberto**
  **Santiago (CL)**
- **COTTET BUSTAMANTE, Luis**
  **Santiago (CL)**
- **HUILIÑIR CURIO, Cesar**
  **Santiago (CL)**
- **CASTILLO NARA, Antonio**
  **Santiago (CL)**

(74) Representative: **Rigamonti, Dorotea Jacobacci & Partners S.p.A. Via Senato, 8 20121 Milano (IT)**

(54) **PAENIBACILLUS POLYMYXA SCHC 33 BACTERIAL STRAIN, AND USE THEREOF TO COMBAT PHYTOPATHOGENIC FUNGI IN FRUITS, VEGETABLES OR PLANTS**

(57)    Biofungicidal composition from a biologically pure culture of a Chilean bacterial isolate obtained from soils of the seventh region of Maule, Chile, corresponding to *Paenibacillus polymyxa* SCHC33, strain with the deposit number RGM2141 granted by the depository authority of the Chilean Collection of Microbial Genetic Resources (CChRGM) to be used as an environmentally friendly, biological control agent against fungal plant diseases, particularly fruits susceptible to infection by *Botrytis cinerea,* efficiently inhibiting conidial germination and mycelium proliferation of said phytopathogenic fungus, furthermore protects plant leaves and fruits from infection by the same fungus, and has the potential to be used in biological control of other fungi and in general of phytopathogenic microorganisms.

Figure 1

**Description**

## FIELD OF THE INVENTION

The present invention discloses a bacterial strain, *Paenibacillus polymyxa* SCHC33, as a biofungicide against phytopathogenic filamentous fungi, particularly against gray mold, *Botrytis cinerea.* This bacterial strain *P. polymyxa* SCHC33 possesses important qualities that will allow it to compete with commercial biofungicides of current use. Its fungicidal activity is very potent and independent of the medium in which the bacterium is cultivated, it is not toxic to humans or plants, it grows rapidly in simple culture media with a Monod kinetic behavior without substrate inhibition, so that it can be produced easily on a large scale and at a low cost. In addition to the above, its ability to sporulate will allow commercial formulations of high stability and long service life.

The field of application of this invention comprises all fruits, vegetables and ornamental plants which are susceptible to infection by phytopathogenic fungi under pre- and post-harvest conditions.

## SUMMARY OF THE INVENTION

This invention provides an isolated wild type bacterial strain, characterized as *Paenibacillus polymyxa* SCHC33, and its use as biofungicide. This bacterial strain was deposited in the Chilean Collection of Microbial Genetic Resources (CChRGM), according to the Budapest treaty for patenting purposes. The deposit number granted on July 23, 2014, by the depository authority is RGM2141, being the depository authority the Chilean Collection of Microbial Genetic Resources (CChRGM). This wild type Chilean native bacterium was isolated from soils in the seventh region of Maule, Chile and has the capacity to kill different isolated and wild type strains of the phytopathogenic fungus *B. cinerea.* The fungicidal capacity of the bacterium is given by the secretion of fungitoxic molecules that interact with the fungus destroying it and as a consequence, inhibiting the germination of conidia and the proliferation of fungal vegetative mycelium.

Many of the biofungicides currently in use could be potentially pathogenic to humans and plants. In contrast, *Paenibacillus polymyxa* SCHC33 has not been reported to be pathogenic or toxic to plants or fruits and has not been reported to be pathogenic to animals or humans. Experiments carried out in the laboratory, reveal that their inoculation in leaves of vines plants does not produce visible alteration in this host after 7 days of incubation at 20°C in plates containing only 1.5% agar-agar (w/v) to maintain humidity at adequate levels. Likewise, when large inocula of the strain [$2 \times 10^{10}$ cfu (colony-forming unit)] are added to intact or wounded grapes, no effect is seen after 7 days of incubation under the same conditions described above.

The biological agent of the invention corresponds to a wild type bacterium, isolated from soils, and has been individualized by biochemical, microbiological, electron microscopy and 16S rDNA sequencing techniques. *P. polymyxa* SCHC33 is a Chilean autochthonous strain and corresponds to a saprophyte organism, non-pathogenic to plants or animals, and therefore the use of its live cells as biofungicide against *B. cinerea* is adequate.

In addition, the present invention relates to compositions containing said bacterial strain or extracts containing the same, or to solutions or mixtures containing compounds derived therefrom (for example, fungitoxic molecules secreted by said bacterial strain), all of which are capable to protect plants and their fruits during the pre- and post-harvest periods, from the attack of phytopathogenic microorganisms.

The present invention also includes any mutant derived from the wild type strain having essentially the same or better properties.

Also, the invention relates to the process for the preparation of said bacterial strain or its derivatives and to the applications thereof in the protection of plants, and in particular the fruits.

In addition, the present invention provides a formulation for controlling fungal plant diseases using the pure strain *P. polymyxa* SCHC33. The use of this bacteria constitutes a natural alternative for synthetic chemical fungicides, ensuring a safer environment to achieve the control or elimination of diseases caused by phytopathogenic fungi.

Finally, the present invention provides a composition containing said strain in an adequate form and amount for its biological activity. The mixture contains non-toxic agents that allow the adherence of the bacterium to the vegetable on which it is inoculated, vegetable nutrients and preservatives in innocuous quantities, in the form of liquid suspension or powder obtained by freeze-drying.

## BACKGROUND OF THE INVENTION

Infections caused by phytopathogenic fungi in any type of plant tissue are extremely devastating, rapidly spreading and occur virtually anywhere on planet earth. The methods currently used to control these diseases are mainly based on the use of chemical fungicides which, despite their potential toxicity, since most of them are recalcitrant molecules, are still applied massively because they allow a relatively efficient control of fungal diseases and because there are still few effective and environmentally safe alternatives.

*Botrytis cinerea* is a phytopathogenic fungus, polyphagous and necrotrophic, infecting plant species of great economic importance, including fruit trees, ornamental plants and vegetables. It produces a disease known as gray rot causing a serious problem pre- and post-harvest in strawberries, raspberries, peaches, apples, pears, chestnuts, kiwi and grapes, among other fruits. In the vine this fungus produces the rot of the bunch of grapes, a disease that is considered at the moment as one of the most serious in the production of export fruits in Chile, since it is capable of causing large losses not only at field level but also during its storage and transportation (Williamson B, Tudzynski B, Tudzynski P, van Kan JA 2007. Botrytis cinerea: the cause of gray mould disease Mol Plant Pathol 8: 561-80; Elad, Y., Williamson B Tudzynski, P. and Delen, N. eds. 2007. Botrytis: Biology, Pathology and Control. The Netherlands: Kluwer Academic Publishers).

At present, the control of this important phytopathogen is mainly carried out with chemical fungicides, however, in recent years some microorganisms with antifungal activity against *B. cinerea* have been described. One of the most promising examples is the Serenade®, whose active ingredient is a bacteria of the genus *Bacillus,* specifically *Bacillus subtilis QST* 713, discovered in soil samples from a vegetable garden by AgraQuest Inc. in Davis, California. This biofungicide is registered in several countries, including Chile, has low toxicity and is being used commercially in Chile and the United States for the control of diseases such as powdery mildew (*Uncinula necator*) and acid rot in vines.

In patent KR20100024454, *Paenibacillus lentimorbus* CMC3723 (deposit number KACC91379) is disclosed, with the ability to suppress pathogens in plants, in particular the pear tree scab (pear scab), *Botrytis* and *Sclerotinum cepivorum,* in addition to preventing the growth of plant pathogens. *Paenibacillus lentimorbus* CMC3723 was isolated from the soil and has the effect of suppressing *Venturia nashicola, Botrytis cinerea, Sclerotium cepivorum* and *Colletotrichum acutatum.* The agent for preventing the diseases caused by these pathogens in plants contains a culture of *Paenibacillus lentimorbus* CMC3723. It is also disclosed a method for isolating, culturing, determining activity against phytopathogenic fungi and bacteria, identifying *Paenibacillus lentimorbus* CMC3723 and preparing a culture medium containing it. In this case, the bacterial strains *Paenibacillus lentimorbus* CMC3723 and *Paenibacillus polymyxa* SCHC33 belong to the same genus, but to totally different species.

Publication KR20090105149 discloses *Paenibacillus polymyxa* NB1 with antibacterial activity and a composition containing it, useful for preventing diseases caused by plant pathogens. *Paenibacillus polymyxa* NB1 acts as an antagonistic agent against plant pathogens and its deposit number is KFCC 11413P. The plant pathogens described are *Colletotrichum acutatum, Pythium ultimum, Phytophthora capsici, Rhizoctonia solani* AG4, *Botrytis cinerea* and *Fusarium oxysporum.*

Patent KR20030075092 teaches a microorganism *Paenibacillus sp.* SD17 that produces antifungal agents for the biological control of plant diseases and a composition for the biological control of plant diseases containing the microorganism for the effective control of diseases in plants. The microorganism *Paenibacillus sp.* SD17 (KCTC10016BP) produces antifungal agents for the biological control of plant diseases including diseases caused by *Pythium spp., Rhizoctonia solani* AG1-1, *Rhizoctonia solani* AG 2-2, *Phytophthora infestans* or *Botrytis cinerea.* A composition for the biological control of plant diseases containing 5 to 30% by weight of the culture medium of *Paenibacillus sp.* SD17 (KCTC-10016BP); 0.2 to 3.0% by weight of an agent which activates spore germination, corresponding to yeast extract; 0.02 to 0.5% by weight of a water-soluble pigment; 1 to 5% by weight of a surfactant and an additive or resin.

Publication EP1241247 discloses antagonistic bacteria for the protection of plants against phytopathogenic bacteria and fungi. The isolated bacteria are of the species *Paenibacillus polymyxa, Pseudomonas chlororaphis, Pseudomonas putida, Serratia plymuthica* and *Bacillus subtilis.* They may be applied as a mixture or separately for the control of, for example, the following plant diseases: crown gill caused by *Agrobacterium tumefaciens* in grapes; diseases caused by *Corynebacterium spp., Pseudomonas syringae* and *Xanthomonas campestris,* in tomato and cabbage. Diseases caused by *Botrytis cinerea* in cucumber and tomato; powdery mildew caused by *Erysiphales spp.* in cucumber and tomato; damping-off caused by *Fusarium oxysporum* in cucumber, melon and tomato; diseases caused by *Helminthosporum sativum* in cereals; the root rot disease caused by *Rhizoctonia solani* in cotton and beans; diseases caused by *Sclerotium rolfsii* in beans; *Dactylium dendroides* in mushrooms and others.

If we compare the antifungal activity, which is obtained in plaque confrontation bioassays against *Botrytis cinerea,* of *Paenibacillus polymyxa* from publication EP1241247 with that of the bacterial strain *Paenibacillus polymyxa* SCHC33 under identical assay conditions, strain SCHC33 produces an inhibition halo of greater diameter and therefore, its fungicidal activity is greater.

## DESCRIPTION OF THE FIGURES

Figure 1 corresponds to scanning electron micrographs of *Paenibacillus polymyxa* SCHC33. In (A) is clearly observed material secreted by the bacteria, corresponding to a biofilm formed by 15 exopolysaccharides. In (B) it is observed grouping of bacilli surrounded by ellipsoidal spores obtained from a liquid culture.

Figure 2 is a transmission electron micrograph of *Paenibacillus polymyxa* SCHC33. There are dividing cells and their corresponding peritrichous flagella.

Figure 3 shows the partial nucleotide sequence of the 16S rDNA of *Paenibacillus polymyxa* SCHC33.

Figure 4 corresponds to confrontation bioassays of *Paenibacillus polymyxa* SCHC33 against *Botrytis cinerea.* In (A), the result of the bioassay performed in a glucose-free minimal medium is shown. In (B), the result of the bioassay performed in a culture medium containing glucose as a carbon source.

In Figure 5, the results of the germination inhibition bioassays of *Botrytis cinerea* conidia on vines leaves are presented. (A) and (B) correspond to the negative controls. (C) and (D) are the positive controls. In (E) and (G) is shown the Serenade® biocontrol effect on germination of conidia for a conidia:bacteria ratio of 1:10 and 1:100, respectively,. In (F) and (H) is observed the biocontrol effect of *Paenibacillus* on germination of conidia for a conidia:bacteria ratio of 1:10 and 1:100, respectively.

Figure 6 shows the bacterial growth curves and glucose consumption for an initial glucose concentration of 2 [g/L]. In (A), the increase of biomass and glucose consumption over time is appreciated. (B) corresponds to the semi-logarithmic curve of biomass over time.

Figure 7 presents the curve of the initial glucose concentration effect on the specific growth rate of 20 *Paenibacillus polymyxa* SCHC33.

Figure 8 shows the experimental growth curve and the fitting to the kinetic models evaluated (Monod, Moser and Tessier models).

In Figure 9 was schematized the region of the 16S rDNA which was amplified by PCR and from which its sequence was obtained. In red primers used for PCR. In blue primers used for sequencing.

Figure 10 shows the 2-liter bioreactor with constant aeration that was used for the determination of the kinetic growth parameters of *Paenibacillus polymyxa* SCHC33.

## DETAILED DESCRIPTION OF THE INVENTION

The strain SCHC33 corresponds to the *polymyxa* species and to the *Paenibacillus* genus. Its characterization by microbiological and biochemical tests is presented in Table 1. It produces colorless/white colonies without pigmentation in potato-dextrose agar (PDA) and in MLG medium (malt extract 10 g/L, glucose 2 g/L, agar-agar 15 g/L).

**Table 1. Morphological and physiological characteristics of *Paenibacillus polymyxa* SCHC33.**

| Shape | Bacillus |
|---|---|
| Gram staining | Positive |
| Motility | (+) |
| Pigment production | (-) |
| Exopolysaccharides production (EPS) | (+) |
| Growth at 4°C | (+) |

**Ultra structural characterization of *Paenibacillus polymyxa* SCHC33 by scanning electron microscopy and transmission electron microscopy**

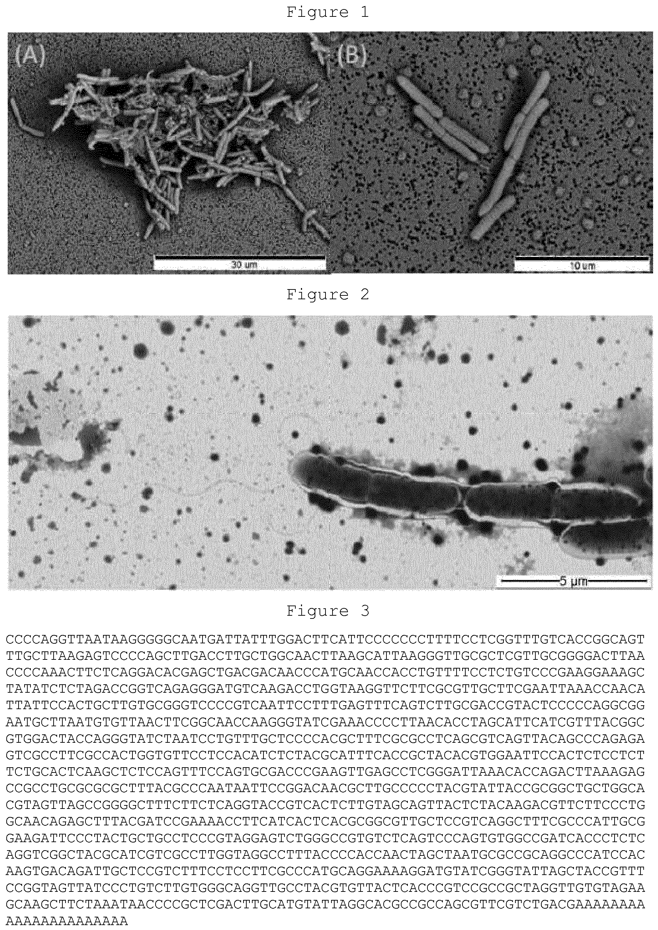

At the level of optical microscopy, the cultures are constituted by Gram-positive mobile bacilli, whereas at the scanning electron microscopy (SEM) level, the bacilli morphology was clearly observed with a cell size in the range of 3 to 5 $\mu$m of length, and 0.5 to 0.8 $\mu$m in diameter (see figure 1). Also, clusters of cells bound by a material of adhesive characteristics corresponding to exopolysaccharides (EPS), which allow the formation of biofilms (see Figure 1A), very important for the adhesion of the bacteria to the surface of the plant to be protected against the attack of phytopathogenic organisms (bacteria or fungi). No bacterial appendages were observed by SEM, however, using the negative staining technique, it was possible to detect under Transmission Electron Microscopy (TEM), the presence of peritrichous flagella of approximately 3 to 5 $\mu$m in length (see figure 2), which agrees perfectly with the background described for this type of bacteria (Lal S. and Tabacchioni S. 2009. Ecology and biotechnological potential of Paenibacillus polymyxa: a minireview, Indian J. Microbiol 49: 2-10). **Molecular characterization of *Paenibacillus polymyxa* SCHC33** In addition to the microbiological and biochemical characterization of the bacteria, the molecular characterization was carried out by obtaining the nucleotide sequence of a portion of the 16S rDNA and its subsequent bioinformatic analysis. In Figure 3 is shown the partial nucleotide sequence of the 16S rDNA of *Paenibacillus polymyxa* SCHC33 (1,255 nucleotides). The comparison of this sequence with existing sequences in databases using BlastN, generated the results shown in Table 2.

**Table 2. Results of the alignment of 16S rDNA of *Paenibacillus polymyxa* SCHC33 on BlastN.** The values of the parameters calculated by the computer program are indicated.

| Accession | Description | Max. Score | Total Score | Cover of the request | E Value | Identity |
|---|---|---|---|---|---|---|
| HE577054 | *Paenibacillus polymyxa* M1 main chromosome, complete genome | 1991 | 27696 | 97% | 0.0 | 98% |
| CP002213 | *Paenibacillus polymyxa* SC2, complete genome | 1991 | 27658 | 97% | 0.0 | 98% |
| EF656457 | *Paenibacillus polymyxa* M-1, partial sequence of the gene of the 16S rRNA | 1991 | 1991 | 97% | 0.0 | 98% |
| AY302439 | *Paenibacillus polymyxa* WY110, partial | 1991 | 1991 | 97% | 0.0 | 98% |
| | sequence of the gene of the 16S rRNA | | | | | |
| JF683620 | *Paenibacillus polymyxa* RS-10, partial sequence of the gene of the 16S rRNA | 1989 | 1989 | 97% | 0.0 | 98% |

Therefore, microbiological tests, the electron microscopy, the 16S rDNA sequencing and bioinformatic analysis confirm that this is a new strain of *Paenibacillus polymyxa,* which was isolated from soils of the Seventh Region of Maule, Chile and was named SCHC33.

**Determination of the antifungal activity of *Paenibacillus polymyxa* SCHC33 against phytopathogenic fungus *Botrytis cinerea***

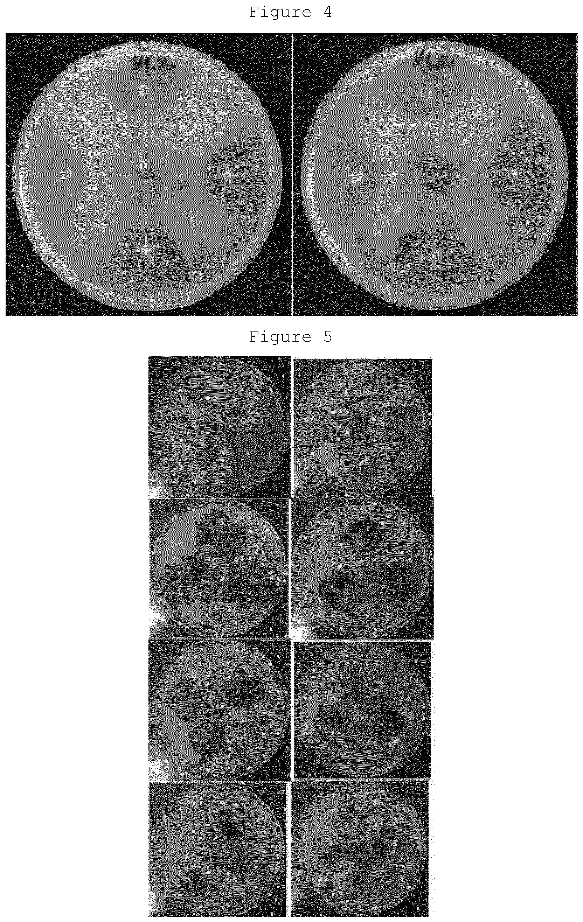

Plaque confronting bioassays were performed in which a fungal mycelium disc was placed in the center of the Petri dish and the bacteria were inoculated at the edges of the same (see Figure 4). The growth inhibition halos of the fungus were clearly observed, both in a glucose-free medium (see Figure 4A) and in a medium containing this monosaccharide (see Figure 4B). In addition if different culture media such as potato-dextrose agar (PDA) are used; Luria Bertani medium containing 0.5% yeast extract, 1% tryptone and 0.5% NaCl; ML medium containing 1.5% malt extract and 0.7% yeast extract, among others, the fungicidal activity remains intact. If in addition to the aforementioned culture media glucose is added, the fungitoxic activity against *B. cinerea* is not altered. This result is very relevant, since it has been observed in other Gram (-) bacteria like *Serratia plymuthica* that the secretion of fungitoxic molecules is repressed in culture media containing glucose. Therefore, for the production of bacterial biomass at industrial scale, any culture medium may be used.

**Determination of the protective capacity of *Paenibacillus polymyxa* SCHC33 against the attack of *Botrytis cinerea* on plant tissue**

Two known amounts of bacteria were used $10^7$ and $10^8$ cfu/mL, in order to evaluate the efficacy of biocontrol compared to *Bacillus subtilis* QST 713, the active component of the commercial bio-fungicide Serenade®.

The results at 7 days were satisfactory, obtaining a slightly superior level of protection of the vegetal tissue with the *Paenibacillus polymyxa* SCHC33 bacterium. The negative control showed small areas of necrosis attributed to the wounds caused in order to facilitate infection and the positive control, in which only conidia of the fungus inoculated, suffered an infection by *Botrytis cinerea* that covered practically 100% of the surface of the leaves. Both in the leaves protected by *Paenibacillus polymyxa* SCHC33 and in those protected by *Bacillus subtilis* QST 713, there were significant decreases in the degree of damage caused by the fungus. In the case of *Paenibacillus polymyxa* SCHC33, using a ratio of conidia:bacteria of 1:10, the damage produced by the fungus only covered 20.5% of the leaf surface, decreasing to 11.7% when the ratio was 1:100. In the trials with *Bacillus subtilis* QST 713, plant tissue necrosis produced by the fungus covered 36.9% of the leaf surface for a ratio of 1:10 and decreased to 15.4% when the ratio was Of 1:100 conidia:bacteria, respectively. These results are shown in Table 3 and Figure 5, showing that the bacterium has a great potential to be used as biofungicide by direct inoculation in the field of Vegetables susceptible to be infected by *Botrytis cinerea.*

**Table 3. Results of damaged surface in vine leaves.**

|  | Total Area [cm$^2$] | Damaged Area [cm$^2$] | Infection |
|---|---|---|---|
| **Control (-) 10$^8$ cfu/mL** | 28,208 | 2,561 | 9,1% |
| **Control (+) 10$^8$ conidia/mL** | 33,625 | 33,296 | 99% |
| ***Bacillus subtilis* QST 713 1:10** | 42,195 | 15,575 | 36,9% |
| ***Bacillus subtilis* QST 713 1:100** | 40,595 | 6,258 | 15,4% |
| ***Paenibacillus polymyxa* SCHC33 1:10** | 35,214 | 7,221 | 20,5% |
| ***Paenibacillus polymyxa* SCHC33 1:100** | 45,596 | 5,340 | 11,7% |

**Determination of the protective capacity of *Paenibacillus polymyxa* SCHC33 against *Botrytis cinerea* attack on 5 grape clusters.**

Protection bioassays against *B. cinerea* were carried out on clusters of grapes of the *Thompson seedless* variety, using 2 known amounts of bacteria, 10$^7$ and 10$^8$ cfu/mL, to evaluate their efficiency as a biocontrol agent. The results at 30 days were of a protection comparable to that shown in vines leaves, since the clusters inoculated with the bacteria by spray did not present evident growth of mycelium of *B. cinerea,* for both amounts of bacteria used. The negative controls did not show *B. cinerea* presence, either in clusters inoculated with only bacteria (10$^8$ cfu/mL) or in those inoculated with sterile water alone. In addition, these results are clear evidence of the innocuousness of the bacteria on the fruit used in the experimentation, since no morphological alteration was observed nor changes in the coloration and neither in the organoleptic characteristics of the grape used. Positive controls (bunches of grapes inoculated by spray only with fungal conidia) showed a clear infection by *B. cinerea,* clearly observing the vegetative growth of the mycelium of the fungus in the berries of inoculated clusters.

**Determination of the kinetic parameters of the growth of *Paenibacillus polymyxa* SCHC33 and adjustment of the results to one of the pre-established models**

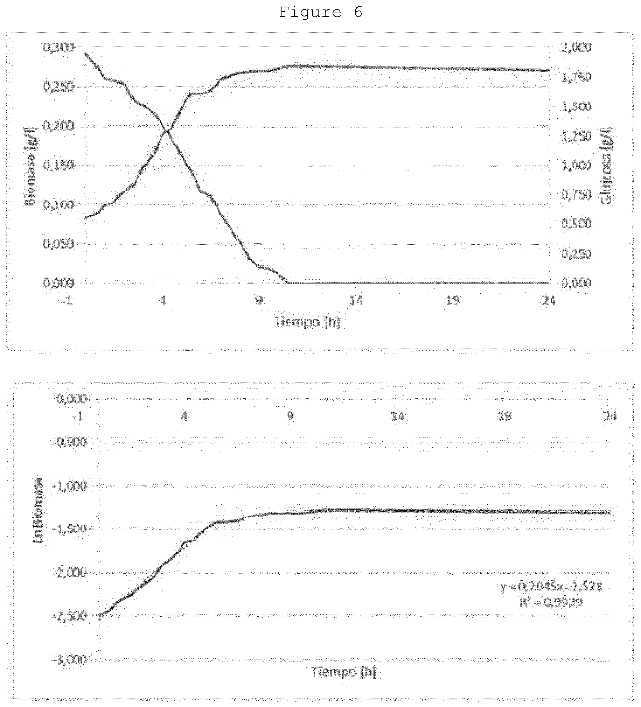

Using a bioreactor of 2 liters capacity, 5 curves of bacterial growth with different concentrations of glucose were obtained. In Table 4, the experimental data obtained are shown, and in Figure 6, the corresponding growth curves for the culture in a medium with 2 g/L glucose are shown, in the first instance the curve that relates the formation of biomass over time and secondly the semi-logarithmic curve used to identify the phases of bacterial growth. Thus it can be seen that there is no latency phase and the exponential growth stage starts immediately at the beginning of the growth, this behavior was repeated in all the experimental runs performed. Glucose consumption occurred at a constant rate throughout the exponential growth stage, achieving in the experiments with a high glucose concentration ($\geq$1 g/L) to be maintained during the period of growth slowdown. No inhibition was observed, either by substrate or product in the bacterial growth, so that the adjustment to kinetic models was restricted to those that do not present this type of situation, in this case Monod, Moser and Tessier (Shuler M., Kargi F. 2002. Stoichiometry of microbial growth and product formation In Bioprocess engineering: Basic concepts, Edited by Shuler M., Kargi F. Harlow: Pearson, 207-218).

**Table 4. Experimental data obtained from biomass increase and glucose consumption for a bacterial growth curve with an initial glucose concentration of 2 [g/L].**

|  | Substrate |  | *Paenibacillus polymyxa* SCHC33 |  |  |
|---|---|---|---|---|---|
| Time [h] | Abs$_{500}$ | Glucose [g/l] | DO$_{600}$ | Biomass [g/l] | ln Biomass |

| | | | | | | |
|---|---|---|---|---|---|---|
| **Exponential phase** | 0 | 0,647 | 1,946 | 0,122 | 0,083 | -2,490 |
| | 0,5 | 0,618 | 1,860 | 0,148 | 0,088 | -2,425 |
| | 1 | 0,576 | 1,735 | 0,198 | 0,099 | -2,310 |
| | 1,5 | 0,570 | 1,718 | 0,225 | 0,105 | -2,254 |
| | 2 | 0,562 | 1,694 | 0,283 | 0,117 | -2,141 |
| | 2,5 | 0,513 | 1,549 | 0,323 | 0,126 | -2,071 |
| | 3 | 0,500 | 1,510 | 0,430 | 0,149 | -1,903 |
| | 3,5 | 0,480 | 1,451 | 0,493 | 0,163 | -1,817 |
| | 4 | 0,443 | 1,341 | 0,621 | 0,190 | -1,660 |
| | 4,5 | 0,402 | 1,220 | 0,665 | 0,200 | -1,612 |
| | 5 | 0,353 | 1,074 | 0,788 | 0,226 | -1,487 |
| | 5,5 | 0,311 | 0,950 | 0,866 | 0,243 | -1,416 |
| **Stationary phase** | 6 | 0,251 | 0,772 | 0,861 | 0,242 | -1,420 |
| | 6,5 | 0,241 | 0,742 | 0,880 | 0,246 | -1,404 |
| | 7 | 0,189 | 0,588 | 0,943 | 0,259 | -1,350 |
| | 7,5 | 0,150 | 0,473 | 0,964 | 0,264 | -1,333 |
| | 8 | 0,106 | 0,342 | 0,988 | 0,269 | -1,313 |
| | 8,5 | 0,060 | 0,206 | 0,992 | 0,270 | -1,310 |
| | 9 | 0,038 | 0,141 | 0,995 | 0,270 | -1,308 |
| | 9,5 | 0,033 | 0,126 | 0,994 | 0,270 | -1,309 |
| | 10 | 0,016 | 0,076 | 1,011 | 0,274 | -1,295 |
| | 10,5 | 0,000 | 0,000 | 1,030 | 0,278 | -1,280 |
| | 24 | 0,000 | 0,000 | 0,998 | 0,271 | -1,305 |

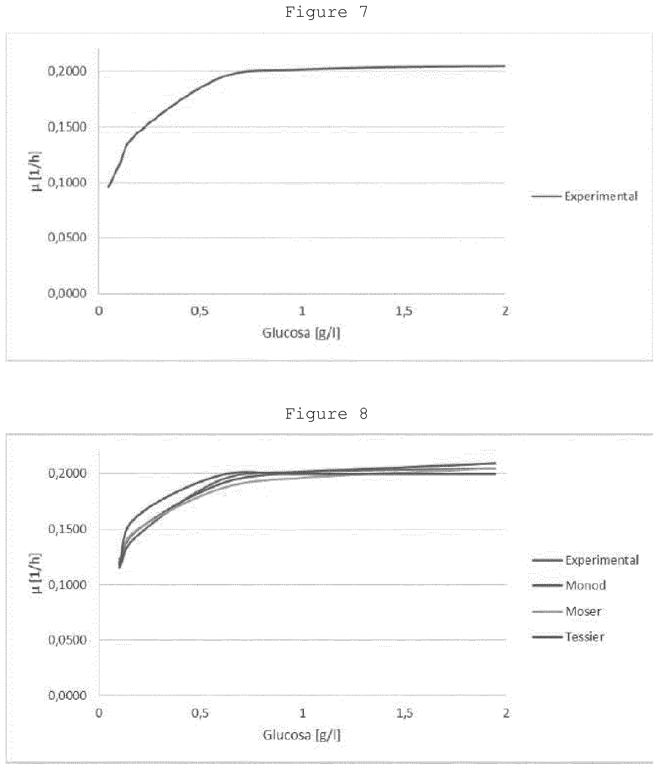

Obtaining the curve that correlates the specific growth rate with the initial concentration of glucose in the medium shown in figure 7, allowed to obtain the intrinsic kinetic parameters of this bacterium growing with glucose as the main substrate. However, the validation of these parameters first requires the statistical validation of the kinetic adjustment to one of the 3 aforementioned models shown in figure 8. In this case the curve obtained presented an expected behavior according to the literature, when increasing the substrate concentration the specific bacterial growth rate continuously increases, until reaching a maximum point from which the rate is constant (Acevedo F., Gentina J. 2004. Cinética de fermentación. In Fundamentos de ingeniería bioquímica. Edited by F. Acevedo, J. Gentina, A. Illanes. Valparaíso: Ediciones universitarias de Valparaiso, 151-168).

The evaluation of the 3 kinetic models was based on the associated statistical parameters as such shown in Table 5, indicated that the growth of *Paenibacillus polymyxa* SCHC33 using glucose as the main substrate fits to a Monod type kinetic model since it is the model with a correlation coefficient closer to 1 and at the same time the model with a lower Chi square parameter, with a difference of one order of magnitude with respect to the other 2 models analyzed. Then the intrinsic kinetic parameters of *Paenibacillus polymyxa* SCHC33 are obtained by analyzing Monod, thus the maximum specific growth rate for this bacterium using Glucose as the main substrate is $\mu max=0.218$ h$^{-1}$, its glucose affinity constant is Ks=0.087 g/L and the yield of biomass production from glucose is YX/S=0.159 [g biomass/g glucose].

**Table 5. Statistical analysis of experimental data obtained and obtaining of the kinetic parameters of the 3 models evaluated.**

| Kinetic | Equation | $\mu_{max}$ | $K_s$ | n | $R^2$ | $X^2$ |
|---|---|---|---|---|---|---|
| Monod | $\mu = \mu_{max} \cdot \dfrac{S}{K_S + S}$ | 0,21 8 | 0,087 | - | 0,996 40 | 0,00 039 |
| Moser | $\mu = \mu_{max} \cdot \dfrac{S^n}{K_S + S^n}$ | 0,21 6 | 0,102 | 0,87 9 | 0,995 00 | 0,00 116 |
| Tessier | $\mu = \mu_{max} \cdot (1 - e^{-S/K_S})$ | 0,19 9 | 0,112 | - | 0,980 82 | 0,00 230 |

## EXPERIMENTAL SECTION

### Obtaining soil samples

Sampling was carried out *in situ* from agricultural soils of the Seventh Region of Maule, About ten random samples, which were taken to the fungi Virology laboratory of the University of Santiago de Chile, where they were stored at room temperature until their posterior utilization.

### Obtaining bacterial isolates

The soil samples were submitted to a heat treatment at 67°C for 48 hours and then 1 gram of each sample was suspended in 1 mL of sterile distilled water. Finally, 1 mL of this suspension for each Sample in triplicate, were inoculated on Petri dishes with MLG+C medium (10 g/L malt extract, 2 g/L glucose, 15 g/L agar-agar, cycloheximide 50 $\mu$g/mL), obtaining diverse microflora from which colonies were isolated and backed up for later analysis.

### Determination of antifungal activity

The various colonies obtained were individually backed up and plaque confrontation bioassays were performed on Petri dishes with MLG medium against *Botrytis cinerea* CCg149, a highly virulent virus-free strain from the fungal Virology laboratory and grown on potato-dextrose agar medium (PDA) until the completion of the tests. Variations in antifungal activity were also evaluated in media of different composition, mainly with and without glucose.
Two types of bio-confrontations were carried out. The first consisted of planting a 5 mm diameter mycelial disk in the center of the Petri dish and at four equidistant points, the same amount of the different bacterial isolates were inoculated and the growth was observed for 7 days at 20°C. The second method consisted of planting 8 pieces of mycelium of 5 mm diameter at equidistant points from the center of the Petri dish, where the bacterial isolate was inoculated. Growth was again observed for 7 days at 20°C. As a control, the same tests were performed by replacing the bacterial isolates with sterile water.
All those bacterial isolates that showed some degree of antifungal activity against the fungus, observable as a halo of inhibition in Petri dishes, were selected.
Subsequently, similar assays were performed using suspension of conidia homogeneously distributed in Petri dishes, which were incubated for 24 hours at 20°C, to ensure the correct adsorption of the sample in the medium. Later, 10 $\mu$L of bacterial culture were inoculated, with an optical density of 0.9 at 600 nm in liquid medium, in the center of the plate and incubated during 7 days at 20°C to observe the germination inhibition halo for *B. cinerea* conidia.

### Obtaining of pure bacterial clones, DNA isolation, amplification of 16S rDNA by PCR and sequencing

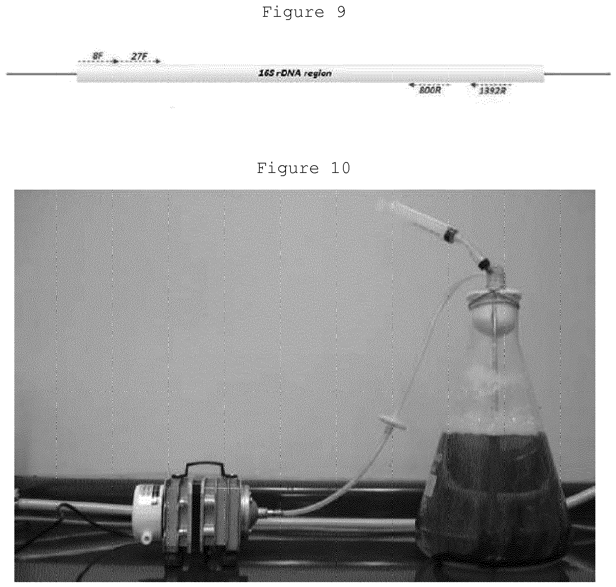

From those bacterial isolates with increased antifungal activity (inhibition halos $\geq$1 cm in Petri dish), serial dilutions were performed to obtain isogenic clones which were considered pure bacterial strains. Genomic DNA of the obtained strains was extracted using the PureLink® Genomic DNA commercial kit and subsequently these samples were subjected to PCR amplification using the eubacterial universal primers designated 8F/1392R (see Figure 9) with the purpose of obtaining a specific 16S rDNA fragment of approximately 1400 base pairs. For PCR, 10 ng of genomic DNA, 0.5 $\mu$M of each primer, 200 $\mu$M dNTPs, 2.5 U of DNA polymerase, 1X reaction buffer and 1.5 mM MgCl$_2$ in a total volume of 50 $\mu$L were used. Cycles consisted of an initial denaturation step of 4.5 minutes at 95°C and 40 cycles of 1 min at 95°C, 1 min at 60°C and 2 min at 72°C, ending in a 5 min at 72°C step. The PCR products were resolved in a 1% agarose (w/v) gel electrophoresis. For the sequencing the primer pair 27F/800R (see figure 9) was used and the sequences obtained were analyzed by BlastN.

### Ultra structural analysis by electron microscopy

In order to obtain images that allowed the determination of morphological and structural aspects of the bacteria, samples of *Paenibacillus polymyxa* SCHC33 were prepared for visualization and analysis by scanning and transmission electron microscopy. For the scanning electron microscope (Jeol JSM-25-SII) samples of liquid bacterial cultures were used, which were prepared with a metallic shading technique using gold. In the case of transmission electron microscopy, negative staining with 1% (w/v) potassium phosphotungstate, pH 7.0, was performed on samples from liquid bacterial cultures and visualized on the Phillips Tecnai 12 Bio Twin microscope at 80 kV.

**Obtaining kinetic parameters**

In order to obtain the kinetic parameters of growth using glucose as the main substrate, experimental runs of discontinuous growth of the bacterium with initial glucose concentrations of 0.1 g/L, 0.2 g/L, 0.5 g/L, 1 g/L, 2 g/L and 5 g/L in LG medium (yeast extract 5 g/L, glucose). Two-liter capacity bioreactors were constructed, as shown in Figure 10, with an air feed sterilized with 2 $\mu$m filters from a 20 L/min capacity compressor and a sample-taker connected to a sterile syringe , with which bacterial culture samples were obtained. In all experimental runs, aeration (1 vvm), temperature (30°C), pH (5) and agitation (200 rpm) were maintained constant at values recommended as optimal by bibliographic data.

For each experimental run 200 mL of bacterial culture in exponential phase of growth were inoculated, in the bioreactor containing 2 liters of culture medium, considering as time 0 the moment when the bioreactor began the agitation and aeration of the sample.

The increase in biomass as the optical density of the bacterial culture at a wavelength of 600 nm was recorded every 30 minutes, and the decrease of the dissolved glucose in the medium was measured using the commercial kit Liquicolor®.

With these data were constructed graphs of bacterial growth and glucose uptake over time, and semi-logarithmic graphs to calculate the specific growth rate ($\mu$) of *Paenibacillus polymyxa* SCHC33, when glucose is used as the main substrate. In addition, the value of the substrate affinity constant ($K_s$) and the biomass yield per substrate ($Y_{x/s}$) were obtained from the same experimental data.

**Adjustment to a kinetic model of bacterial growth**

To adjust the growth of *Paenibacillus polymyxa* SCHC33 to one of the bacterial growth kinetic models, the specific growth rate values obtained in each experimental run were used and a graph was constructed which relates the initial concentrations of glucose to the specific growth rates, obtained from the measurements. Three bacterial growth kinetics were evaluated where the different mathematical models adjusted to the experimental data varying one or more constants, depending on the model, which are estimated minimizing, by Newton's method, the residual sum of squares (RSS) between the experimental values and those calculated using the Microsoft Office add-on Excel *Solver.* To evaluate which model is the one that presented a better fit to the experimental data the following statistical parameters were used:

$$\text{Correlation coeficient} \qquad R^2 = \frac{\sum_{i=i}^{N}(V_{cal} - V_{exp})^2}{\sum_{i=1}^{N}(V_{exp} - V_{cal})^2} \ (4-3)$$

where:

$V_c$: Calculated value based on the model
$V_e$: Experimental values
$N$ : Data number

$$\text{Chi squared} \qquad \chi^2 = \frac{RSS}{N - n} \qquad (4-2)$$

where:

*RSS* : Residual sum of squares

*N* : Data number

*n* : Constant number

**Bioassays in plant tissue**

Inhibition of conidia germination on plant tissue was observed, using leaves of vines harvested immediately before use. The leaves were washed with a solution of sodium hypochlorite 0.5% (v/v) and then with sterile distilled water. Subsequently, they were incubated in Petri dishes with 1.5% (w/v) agar-agar to maintain moisture during the 7-day duration of the assay. The leaves were wounded to facilitate infection and then inoculated with bacterial culture in liquid medium and suspension of conidia in proportions of 1:10 and 1:100, respectively. As a control, leaves inoculated only with conidia and leaves inoculated only with bacteria were prepared, in addition to a control consisting of leaves inoculated only with sterile water and a control using the active principle of the commercial biofungicide named Serenade®, *Bacillus subtilis* QST 713 (Table 6).

**Table 6. Experimental treatments performed.**

| Treatment | Conidia suspension | *Paenibacillus polymyxa* SCHC33 culture | Distilled H₂O | *Bacillus subtilis* QST 713 (Serenade®) |
|---|---|---|---|---|
| **1** | + | - | + | - |
| **2** | + | + | - | - |
| **3** | + | - | - | + |
| **4** | - | + | - | - |
| **5** | - | - | + | - |
| **6** | - | - | - | + |

The results were quantified as percentage of leaf area damaged with respect to the total surface at 7 days of incubation at 20°C. For this purpose, ImageJ software (http://rsb.info.nih.gov/ij/index.html) was used to obtain the respective areas, all determined after 7 days of incubation at 20 °C.

**Determination of the protective capacity of *Paenibacillus polymyxa* SCHC33 against Botrytis cinerea attack on grape clusters.**

The inhibition of the conidia germination on fruits was observed, using clusters of *Thompson seedless* grapes. The clusters were washed with a solution containing 0.5% (v/v) sodium hypochlorite and then with sterile distilled water, then incubated in disinfected closed containers for the 30 days of duration of the assay. The clusters were inoculated with suspensions of conidia and bacteria in proportions of 1:10 and 1:100 (conidia:bacteria). As controls, clusters were inoculated only with conidia, others only with bacteria and a control consisting of clusters inoculated only with sterile distilled water (Table 7).

**Table 7. Experimental treatments performed.**

| Treatment | Conidia suspension | *Paenibacillus* culture | Distilled H₂O |
|---|---|---|---|
| **1** | + | + | - |
| **2** | + | + | - |
| **3** | + | - | - |
| **4** | - | + | - |
| **5** | - | - | + |

The results were analyzed qualitatively, determining the presence or absence of *Botrytis cinerea* mycelial growth on the surface of the clusters. Observations were made during the 30-day period of incubation at 20°C.

**Claims**

1.   Strain of the bacterial species *Paenibacillus polymyxa* SCHC33, wherein it is the strain with the deposit number RGM2141 granted by the Chilean Collection of Microbial Genetic Resources (CChRGM) depository authority.

2.   The strain of claim 1, wherein it comprises a high fungicidal activity which is independent of the components of the medium which are used to cultivate the strain.

3.   The strain of claim 1 or 2, wherein it grows according to bacterial growth kinetics that fits to the Monod model, which allows a rapid increase of the cellular biomass, without any inhibition (by substrate or product), keeping the fungicidal activity intact.

4. The strain of any of claims 1 to 3, wherein the strain is psychrotrophic, growing in a temperature range of 4°C to 40°C, with an optimal growth temperature of 30°C.

5. The strain of any of claims 1 to 4, wherein the strain grows in a pH range of 3 to 10, with an optimum pH equal to 5.0.

6. A biofungicidal extract, wherein it corresponds to an active derivative of the strain of claim 1, maintaining the same high fungicidal activity as said strain.

7. A biofungicidal composition, wherein it comprises the strain according to claim 1 and an acceptable agricultural vehicle.

8. The biofungicidal composition of claim 7 wherein it comprises $10^5$-$10^8$ cfu/ml of the strain.

9. The biofungicidal composition of claim 8, wherein it comprises vegetative cells or spores suspended in aqueous solution.

10. A biofungicidal composition, wherein it comprises the extract according to claim 6; and an acceptable agricultural vehicle.

11. Use of the strain according to any of claims 1 to 5, wherein it serves to prepare a composition useful for the control of fungal infections caused by phytopathogenic fungi in plants, fruits or vegetal tissue.

12. Use according to claim 12, wherein said fungal infection is produced by a phytopathogenic fungus belonging to the genus *Botrytis.*

13. Use according to claim 11, wherein said fruit is in a pre-harvest state, post-harvest, or in a state of storage, transfer or conservation.

14. A method for preventing fruit rot, wherein it comprises spraying on the fruits a biofungicidal composition according to any one of claims 7 to 9, or according to claim 10.

15. The method according to claim 14, wherein said spray is liquid cultures spraying.

16. A method for preventing fruit rot, wherein it comprises spaying the fruits with a biofungicidal composition according to claim 10.

Figure 1

Figure 2

Figure 3

CCCCAGGTTAATAAGGGGGCAATGATTATTTGGACTTCATTCCCCCCCTTTTCCTCGGTTTGTCACCGGCAGT
TTGCTTAAGAGTCCCCAGCTTGACCTTGCTGGCAACTTAAGCATTAAGGGTTGCGCTCGTTGCGGGGACTTAA
CCCCAAACTTCTCAGGACACGAGCTGACGACAACCCATGCAACCACCTGTTTTCCTCTGTCCCGAAGGAAAGC
TATATCTCTAGACCGGTCAGAGGGATGTCAAGACCTGGTAAGGTTCTTCGCGTTGCTTCGAATTAAACCAACA
TTATTCCACTGCTTGTGCGGGTCCCCGTCAATTCCTTTGAGTTTCAGTCTTGCGACCGTACTCCCCCAGGCGG
AATGCTTAATGTGTTAACTTCGGCAACCAAGGGTATCGAAACCCCTTAACACCTAGCATTCATCGTTTACGGC
GTGGACTACCAGGGTATCTAATCCTGTTTGCTCCCCACGCTTTCGCGCCTCAGCGTCAGTTACAGCCCAGAGA
GTCGCCTTCGCCACTGGTGTTCCTCCACATCTCTACGCATTTCACCGCTACACGTGGAATTCCACTCTCCTCT
TCTGCACTCAAGCTCTCCAGTTTCCAGTGCGACCCGAAGTTGAGCCTCGGGATTAAACACCAGACTTAAAGAG
CCGCCTGCGCGCGCTTTACGCCCAATAATTCCGGACAACGCTTGCCCCCTACGTATTACCGCGGCTGCTGGCA
CGTAGTTAGCCGGGGCTTTCTTCTCAGGTACCGTCACTCTTGTAGCAGTTACTCTACAAGACGTTCTTCCCTG
GCAACAGAGCTTTACGATCCGAAAACCTTCATCACTCACGCGGCGTTGCTCCGTCAGGCTTTCGCCCATTGCG
GAAGATTCCCTACTGCTGCCTCCCGTAGGAGTCTGGGCCGTGTCTCAGTCCCAGTGTGGCCGATCACCCTCTC
AGGTCGGCTACGCATCGTCGCCTTGGTAGGCCTTTACCCCACCAACTAGCTAATGCGCCGCAGGCCCATCCAC
AAGTGACAGATTGCTCCGTCTTTCCTCCTTCGCCCATGCAGGAAAAGGATGTATCGGGTATTAGCTACCGTTT
CCGGTAGTTATCCCTGTCTTGTGGGCAGGTTGCCTACGTGTTACTCACCCGTCCGCCGCTAGGTTGTGTAGAA
GCAAGCTTCTAAATAACCCCGCTCGACTTGCATGTATTAGGCACGCCGCCAGCGTTCGTCTGACGAAAAAAAA
AAAAAAAAAAAAAAA

Figure 4

Figure 5

Figure 6

Figure 7

Figure 8

Figure 9

Figure 10

## INTERNATIONAL SEARCH REPORT

| | International application No. |
|---|---|
| | PCT/CL2015/050031 |

**A. CLASSIFICATION OF SUBJECT MATTER**

C12N1/20; A01N63/00 (2015.01)

According to International Patent Classification (IPC) or to both national classification and IPC

**B. FIELDS SEARCHED**

Minimum documentation searched (classification system followed by classification symbols)

(CIP): C12N1/20; A01N63/00

Documentation searched other than minimum documentation to the extent that such documents are included in the fields searched

Electronic data base consulted during the international search (name of data base and, where practicable, search terms used)

GOOGLE PATENT, GOOGLE ACADEMIC, BEIC, EPOQUE

**C. DOCUMENTS CONSIDERED TO BE RELEVANT**

| Category* | Citation of document, with indication, where appropriate, of the relevant passages | Relevant to claim No. |
|---|---|---|
| X | CN 101519639 B (RES CT ECO ENVIRONMENTAL SCIEN) 13.04.2011 Paragraphs 13, 31, 33. | 1, 3, 6, 7, 10-12 |
| Y | | 2, 4-5, 8-9, 13-16 |
| Y | SHARMA, J.K. et al. Isolation and identification of proteolytic psychrotrophic sporeforming bacteria from raw milk supplies at an experimental dairy in India. Journal of the society of Daily technology: Volume 37 No. 3 July 1984. p. 96-98. page 97. | 2, 4 |
| Y | US 7935335 B2 (KAKEN PHARM. CO LTDA) 03.05.2011 column 12 (Líneas 17-27). | 8-9, 13-16 |
| Y | ABIS ENCYCLOPEDY, [publicación en línea] fecha de actualización 30/11/2012 (determinado en www.archives.org), retrieved on 02-12-2015 from internet: http://www.tgw1916.net/Bacillus/polymyxa.html the whole document | 4-5 |

| X | Further documents are listed in the continuation of Box C. | X | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04/09/2015 | 04/12/2015 |

| Name and mailing address of the ISA/ INAPI, Av. Libertador Bernardo O'Higgins 194, Piso 17, Santiago, Chile | Authorized officer NARVAEZ H., Claudio |
|---|---|
| Facsimile No. | Telephone No. Nº de teléfono 56-2-28870551 56-2-28870550 |

Form PCT/ISA/210 (second sheet) (January 2015)

**INTERNATIONAL SEARCH REPORT**

International application No.

PCT/CL2015/050031

C (Continuation). DOCUMENTS CONSIDERED TO BE RELEVANT

| Category* | Citation of document, with indication, where appropriate, of the relevant passages | Relevant to claim No. |
|---|---|---|
| A | HENRIQUEZ, J.L. et al. Inhibitory effects of Bacillus amyloliquefasciens and Paenibacillus polymyxa on Botrytis cinerea causing gray rot of grapes. APS IPPC joint Meeting. 6-10 August 2011. Abstract | |
| A | LIU, J. et al. Medium optimization and estructural characterization of exopolysaccharides from endophytic bacterium Paenibacillus polymyxa EJS-3. Carbohydrate Polymers 79 (2010), 206-2013. doi: 10.1016/j.carbpol.2009.07.055 Page 206. | |
| A | LING, N., et al. Identification and role of organic acids in watermelon root exidates for recruiting Paenibacillus polymyxa SQR-21 in the rhizosphere. European Journal of Soil Biology 47 (2011), 374-379. doi: 10.1016/j.ejsoby.2011.08.009 The whole document | |

Form PCT/ISA/210 (continuation of second sheet) (January 2015)

## INTERNATIONAL SEARCH REPORT

| International application No. |
| --- |
| PCT/CL2015/050031 |

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  [X]  forming part of the international application as filed:

        [ ]  in the form of an Annex C/ST.25 text file.

        [X]  on paper or in the form of an image file.

    b.  [ ]  furnished together with the international application under PCT Rule 13*ter*.1(a) for the purposes of international search only in the form of an Annex C/ST.25 text file.

    c.  [ ]  furnished subsequent to the international filing date for the purposes of international search only:

        [ ]  in the form of an Annex C/ST.25 text file (Rule 13*ter*.1(a)).

        [ ]  on paper or in the form of an image file (Rule 13*ter*.1(b) and Administrative Instructions, Section 713).

2.  [ ]  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet (1)) (January 2015)

**INTERNATIONAL SEARCH REPORT**
Information on patent family members

International application No.

PCT/CL2015/050031

| | | | |
|---|---|---|---|
| CN101519639 B | 13.04.2011 | NONE | |
| US 7935335 B2 | 03.05.2011 | EP1788074 (B1) | 23.05.2007 |
| | | ES2379178 (T3) | 23.04.2012 |
| | | JP4359619 (B2) | 04.11.2009 |
| | | KR101227714 (B1) | 22.05.2007 |
| | | MX2007001335 (A) | 04.07.2007 |
| | | WO2006016558 (A1) | 16.02.2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20100024454
- KR 20090105149
- KR 20030075092
- EP 1241247 A

### Non-patent literature cited in the description

- **WILLIAMSON B ; TUDZYNSKI B ; TUDZYNSKI P ; VAN KAN JA.** Botrytis cinerea: the cause of gray mould disease. *Mol Plant Pathol,* 2007, vol. 8, 561-80
- Botrytis: Biology, Pathology and Control. The Netherlands: Kluwer Academic Publishers
- **LAL S. ; TABACCHIONI S.** Ecology and biotechnological potential of Paenibacillus polymyxa: a minireview. *Indian J. Microbiol,* 2009, vol. 49, 2-10
- Stoichiometry of microbial growth and product formation. **SHULER M. ; KARGI F.** Bioprocess engineering: Basic concepts. 2002, 207-218
- Cinética de fermentación. **ACEVEDO F. ; GENTINA J.** Fundamentos de ingeniería bioquímica. 2004, 151-168